(19) Europäisches Patentamt
European Patent Office
Office européen des brevets

(11) Veröffentlichungsnummer : **0 362 707 B1**

# (12) EUROPÄISCHE PATENTSCHRIFT

(45) Veröffentlichungstag der Patentschrift :
**13.05.92 Patentblatt 92/20**

(51) Int. Cl.$^5$ : **C07C 217/36,** C07C 213/00, A61K 31/23

(21) Anmeldenummer : **89118046.5**

(22) Anmeldetag : **29.09.89**

(54) **Hydroxypropafenonglyceride.**

(30) Priorität : **07.10.88 DE 3834132**

(43) Veröffentlichungstag der Anmeldung :
**11.04.90 Patentblatt 90/15**

(45) Bekanntmachung des Hinweises auf die Patenterteilung :
**13.05.92 Patentblatt 92/20**

(84) Benannte Vertragsstaaten :
**AT BE CH DE ES FR GB IT LI NL**

(56) Entgegenhaltungen :
**EP-A- 0 075 207**
**EP-A- 0 077 529**
**CHEMICAL ABSTRACTS, Band 104, Nr. 23, 9. Juni 1986, Columbus, Ohio, USA; H.G. HEGE et al. "Studies on the metabolism ofpropafenone. 3rd Comm.: Isolation of the conjugated metabolites in the dog and identification using fast atom bombardment massspectrometry", Spalte 199506, Zusammenfassung-Nr. 199 504m**

(73) Patentinhaber : **KNOLL AG**
**Knollstrasse 32**
**W-6700 Ludwigshafen (DE)**

(72) Erfinder : **Rosenberg, Joerg, Dr.**
**Tiroler Strasse 6**
**W-6700 Ludwigshafen (DE)**
Erfinder : **Heberger, Juergen**
**Ebertstrasse 9**
**W-6707 Schifferstadt (DE)**
Erfinder : **Gruenhagen Hans-Heinrich, Dr.**
**In den Weihergaerten 59**
**W-6700 Ludwigshafen (DE)**
Erfinder : **Brode, Egon, Dr.**
**Max-Liebermann-Strasse 10 b**
**W-6710 Frankenthal (DE)**
Erfinder : **Von Philipsborn, Gerda, Dr.**
**Naechstenbacher Weg 35**
**W-6940 Weinheim (DE)**

(74) Vertreter : **Höller, Klaus, Dr. et al**
**BASF Aktiengesellschaft Carl-Bosch-Strasse 38**
**W-6700 Ludwigshafen (DE)**

EP 0 362 707 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue Hydroxypropafenonglyceride, deren Herstellung und Verwendung als Wirkstoffe für Antiarrhythmika.

Viele Wirkstoffe können aufgrund ihres hydrophilen Charakters nur bedingt aus dem Gastrointestinaltrakt durch die Zellen der Darmwand resorbiert werden. Durch Lipophilisierung solcher Wirkstoffe mit Hilfe eines unter physiologischen Bedingungen leicht wieder abspaltbaren Glyceridrests kann es gelingen, die Resorption hydrophiler Wirkstoffe durch Membranen hindurch zu verbessern. Auch der bei manchen hydrophilen Wirkstoffen ausgeprägte "first-pass"-Effekt kann umgangen werden, da diese lipophilen Wirkstoffderivate oftmals über die Lymphe resorbiert werden und somit die erste Leberpassage umgehen. Die Lipophilisierung kann z.B. mit dem in der EP-A 77 529 angegebenen Verfahren erfolgen, indem Glyceridbausteine mit Hilfe einer als Brückenglied fungierenden Dicarbonsäure mit funktionellen Gruppen des Wirkstoffs umgesetzt werden. Es zeigte sich dann aber, daß es z.B. mit dem nach dem Verfahren dieser EP-A hergestellten Derivat des Pindolols nicht gelang, nach peroraler Gabe am Hund bei gleicher molarer Wirkstoffmenge mit dem lipophilen Derivat höhere Plasmakonzentrationen an Pindolol zu erhalten als nach Gabe des Wirkstoffs selbst (Acta Pharm. Suec. 23 (1986), 163-172). Auch der Versuch, die Resorption des hydrophilen Wirkstoffs Heparin aus dem Gastrointestinaltrakt durch Lipophilisierung mit dem obigen Verfahren zu verbessern, gelang nicht (T. Albig, Dissertation ETH Zürich Nr. 7990 (1986)).

Es wurde nun gefunden, daß sich die Bioverfügbarkeit zweier Wirkstoffe durch Glyceridbindung stark verbessern läßt.

Gegenstand der Erfindung sind Hydroxypropafenonglyceride der Formel I

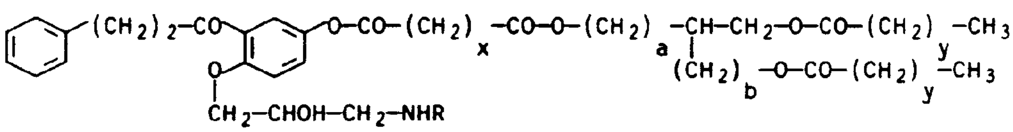

I,

worin

R ein n-Propyl- oder 1,1-Dimethylpropylrest,
x die Zahl 2 oder 3 und
y die Zahl 12, 14, 16, 18 oder 20 bedeuten und
a und b voneinander verschieden sind und 0 oder 1 bedeuten,

sowie deren Stereoisomere.

Die neuen Verbindungen besitzen zwei asymmetrische C-Atome und können daher in Form von Stereoisomeren vorliegen.

Die neuen Verbindungen lassen sich herstellen, indem man eine Verbindung der Formel II

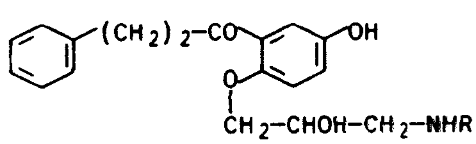

II,

worin R die angegebene Bedeutung hat, mit einem Glycerid der Formel III

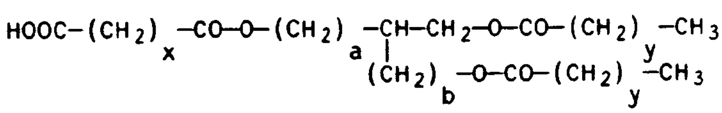

III,

worin x, y, a und b die angegebene Bedeutung besitzen, umsetzt.

Für die Umsetzung der Verbindung II mit III muß zunächst die NH-Gruppe in II geschützt werden. Das kann mit Hilfe einer Aminoschutzgruppe, wie Chlorameisensäurebenzylester geschehen. Die Einführung der Schutzgruppe erfolgt nach in der Peptidchemie bekannten Methoden, z.B. in einem Zweiphasengemisch aus Diethylether und Wasser.

Danach wird die so erhaltene Verbindung mit III verestert, was wiederum nach bekannten Methoden

geschehen kann, beispielsweise durch Überführen von III in das Säurechlorid mit $SOCl_2$ und Umsetzen des so erhaltenen Säurechlorids mit der an der Aminogruppe geschützten Verbindung II in Methylenchlorid/Pyridin bei Raumtemperatur.

Die Veresterung erfolgt überraschenderweise hochselektiv an der phenolischen Hydroxygruppe. Die Hydroxygruppe an der aliphatischen Seitenkette bleibt dabei frei und muß nicht mit einer eigenen OH-Schutzgruppe versehen sein.

Nach der Veresterung wird die Schutzgruppe in bekannter Weise abgespalten, beispielsweise durch Hydrieren mit $Pd/H_2$ bei Normaldruck in Ethylmethylketon.

Die für die Umsetzung benötigten Ausgangsstoffe der Formel II sind bekannt (DE-C 2 001 431, EP-A 75 207 (Beispiel 71)).

Zur Herstellung der Stereoisomeren geht man von den optisch aktiven Vorstufen aus.

Durch die oben beschriebene Derivatisierung wird die Bioverfügbarkeit der Verbindungen II, die nach peroraler Gabe lediglich bei etwa 5 % liegt, um den Faktor 4 bis 5 erhöht, d.h. die eingesetzte Wirkstoffmenge kann um diesen Faktor reduziert werden.

Dieser Befund ist insofern überraschend, als es bekannt ist, daß man nach dem angegebenen Verfahren mit Pindolol und Heparin keine Verbesserung der enteralen Verfügbarkeit erreicht. Auch mit Propafenon erreicht man auf diese Weise keine verbesserte enterale Verfügbarkeit.

Die Verbindungen I eignen sich wie die Verbindungen II zur Therapie und Prophylaxe von ventrikulären Extrasystolen, ventrikulären Tachykardien und Tachyarrhythmien, supraventrikulären Extrasystolen, Tachykardien und Tachyarrhythmien, sowie zur Prophylaxe von Kammerflimmern nach Elektrokonversion.

Die folgenden Beispiele veranschaulichen die Erfindung.

Beispiel 1

a) Herstellung von N-Benzyloxycarbonyl-5-hydroxypropafenon

321,7 g (0,9 mol) 5-Hydroxypropafenon (Base) wurden in einer Mischung aus 1800 ml Diethylether und 1800 ml Wasser zusammen mit 112,5 g Magnesiumoxid suspendiert. Unter Eiskühlung tropfte man dann unter kräftigem Rühren 307,1 g (0,9 mol) einer 50 %igen Chlorameisensäurebenzylesterlösung (in Toluol) zu. Man gab nach 2 h weitere 15 ml dieser Lösung zu. Nach 6 h wurde mit 3000 ml Essigsäureethylester versetzt und so lange konzentrierte Salzsäure zugetropft, bis eine klare Lösung entstand. Die abgetrennte organische Phase wurde nach Filtration mit 1000 ml Salzsäure (1 molar) und mit Wasser (1000 ml) gewaschen, über Natriumsulfat getrocknet und schließlich eingedampft. Den öligen Rückstand versetzte man mit 2000 ml Diisopropylether und ließ unter Rühren kristallisieren. Nach dem Absaugen wurden die Kristalle mit wenig Diisopropylether gewaschen und schließlich im Vakuum getrocknet.
Ausbeute: 413 g (93 %)
Schmelzpunkt: 84-86°C

b) Veresterung von N-Benzyloxycarbonyl-5-hydroxypropafenon mit 1,2-Dipalmitoyl-glycero-3-(3-(chlorformyl)propionat))

235,6 g (0,48 mol) N-(Benzyloxycarbonyl)-5-hydroxypropafenon wurden in 2500 ml trockenem Dichlormethan gelöst. Den Ansatz kühlte man unter starkem Rühren auf -10°C Innentemperatur ab. In diese Mischung tropfte man aus getrennten Tropftrichtern gleichzeitig die folgenden Lösungen so zu, daß beide Tropftrichter am Ende zur gleichen Zeit leer waren:

– Lösung 1: 330 g (0,48 mol) 1,2-Dipalmitoylglycero-3-(3-chlorformyl)-propionat, gelöst in 800 ml Dichlormethan;
– Lösung 2: 48,4 g (0,48 mol) Triethylamin, gelöst in 400 ml Dichlormethan.

Nach beendeter Zugabe ließ man die Temperatur der Reaktionslösung langsam auf Raumtemperatur ansteigen. Der Ansatz wurde dann zweimal mit je 1200 ml Salzsäure (1 molar) und schließlich mit 1200 ml Wasser ausgeschüttelt, über Natriumsulfat getrocknet und eingedampft. In den noch warmen öligen Rückstand goß man unter Rühren 4000 ml Methanol, kühlte unter Rühren im Eisbad, saugte den ausgefallenen Niederschlag bei 8°C ab und trocknete im Vakuum.
Ausbeute: 521 g (95 %)
Schmelzpunkt: 34-36°C.

c) Herstellung des Endproduktes

342,3 g (0,3 mol) des gemäß b) erhaltenen Vorprodukts wurden in 2000 ml Aceton gelöst und mit 180 ml Salzsäure (2 molar) versetzt. Man spülte dann die Apparatur mit Stickstoff, gab 30 g Palladium-Kohle (10 %ig) zu und hydrierte bei 40°C unter Normaldruck mit Wasserstoffgas. Das entstehende Kohlendioxid wurde mit Natronlauge abgefangen. Nach 1 h Hydrierung wurde die Kohle abgesaugt, mit Aceton kurz gewaschen und das Filtrat ließ man unter Rühren im Eisbad kristallisieren, saugte dann den Niederschlag im Kälteraum ab, wusch mit wenig kaltem Aceton nach und trocknete schließlich im Vakuum. Man erhielt so die Verbindung I, R = n-Propyl, x = 2, y = 14, a = 1, b = 0.

Ausbeute: 291 g (91 %)

Schmelzpunkt: 50-51°C

Analog Beispiel 1 wurden hergestellt:

2. Verbindung I, R = n-Propyl, x = 2, y = 12, a = 1; b = 0

Ausbeute: 63 %

Schmelzpunkt: < 30°C

Bei der Herstellung des Endproduktes war die Hydrierung nach 2 h beendet, und die Kristallisation erfolgte nicht mit Aceton sondern in Acetonitril-Methanol 95:5.

3. Verbindung I, R = n-Propyl, x = 2, y = 14, a = 0, b = 1

Ausbeute: 78 %

Schmelzpunkt: 39-41°C

Verwendet man in den Beispielen 1 bis 3 optisch aktive Ausgangsstoffe II oder III, so erhält man die Verbindungen I in optisch aktiver Form, setzt man II und III als Antipoden ein, so erhält man die entsprechenden R,R-, R,S-, S,R- und S,S-Enantiomeren.

Analog lassen sich die in der folgenden Tabelle angeführten Verbindungen der Formel I herstellen, und zwar sowohl als Racemate als auch in Form ihrer Antipoden bzw. ihrer R,R-, R,S-, S,R- und S,S-Enantiomeren.

Tabelle

| R | x | y | a | b |
|---|---|---|---|---|
| n-Propyl | 2 | 12 | 1 | 0 |
| n-Propyl | 2 | 14 | 1 | 0 |
| n-Propyl | 2 | 16 | 1 | 0 |
| n-Propyl | 2 | 18 | 1 | 0 |
| n-Propyl | 2 | 20 | 1 | 0 |
| 1,1-Dimethylpropyl | 2 | 12 | 1 | 0 |
| 1,1-Dimethylpropyl | 2 | 14 | 1 | 0 |
| 1,1-Dimethylpropyl | 2 | 16 | 1 | 0 |
| 1,1-Dimethylpropyl | 2 | 18 | 1 | 0 |
| 1,1-Dimethylpropyl | 2 | 20 | 1 | 0 |
| n-Propyl | 3 | 12 | 1 | 0 |
| n-Propyl | 3 | 14 | 1 | 0 |
| n-Propyl | 3 | 16 | 1 | 0 |
| n-Propyl | 3 | 18 | 1 | 0 |
| n-Propyl | 3 | 20 | 1 | 0 |
| 1,1-Dimethylpropyl | 3 | 12 | 1 | 0 |
| 1,1-Dimethylpropyl | 3 | 14 | 1 | 0 |
| 1,1-Dimethylpropyl | 3 | 16 | 1 | 0 |
| 1,1-Dimethylpropyl | 3 | 18 | 1 | 0 |
| 1,1-Dimethylpropyl | 3 | 20 | 1 | 0 |
| n-Propyl | 2 | 12 | 0 | 1 |
| n-Propyl | 2 | 14 | 0 | 1 |
| n-Propyl | 2 | 16 | 0 | 1 |
| n-Propyl | 2 | 18 | 0 | 1 |
| n-Propyl | 2 | 20 | 0 | 1 |
| 1,1-Dimethylpropyl | 2 | 12 | 0 | 1 |
| 1,1-Dimethylpropyl | 2 | 14 | 0 | 1 |
| 1,1-Dimethylpropyl | 2 | 16 | 0 | 1 |
| 1,1-Dimethylpropyl | 2 | 18 | 0 | 1 |
| 1,1-Dimethylpropyl | 2 | 20 | 0 | 1 |
| n-Propyl | 3 | 12 | 0 | 1 |
| n-Propyl | 3 | 14 | 0 | 1 |
| n-Propyl | 3 | 16 | 0 | 1 |
| n-Propyl | 3 | 18 | 0 | 1 |
| n-Propyl | 3 | 20 | 0 | 1 |
| 1,1-Dimethylpropyl | 3 | 12 | 0 | 1 |
| 1,1-Dimethylpropyl | 3 | 14 | 0 | 1 |
| 1,1-Dimethylpropyl | 3 | 16 | 0 | 1 |
| 1,1-Dimethylpropyl | 3 | 18 | 0 | 1 |
| 1,1-Dimethylpropyl | 3 | 20 | 0 | 1 |

## Patentansprüche

1. Hydroxypropafenonglyceride der Formel I

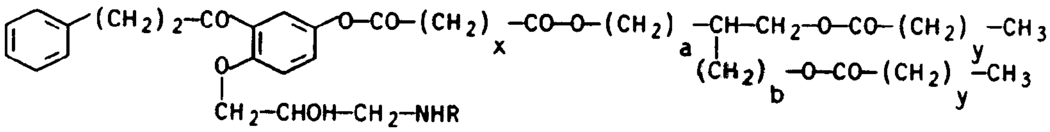
I,

worin

R ein n-Propyl- oder 1,1-Dimethylpropylrest,
x die Zahl 2 oder 3,
y die Zahl 12, 14, 16, 18 oder 20 bedeuten und
a und b voneinander verschieden sind und 0 oder 1 bedeuten,

sowie deren Stereoisomere.

2. Verfahren zur Herstellung der Hydroxypropafenonglyceride der Formel I

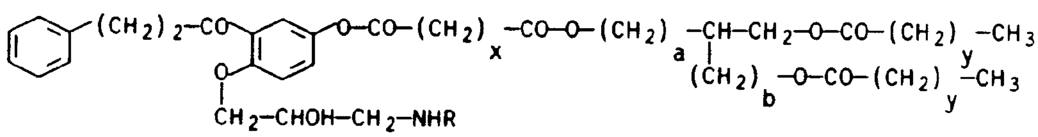
I,

worin

R ein n-Propyl- oder 1,1-Dimethylpropylrest,
x die Zahl 2 oder 3,
y die Zahl 12, 14, 16, 18 oder 20 bedeuten und
a und b voneinander verschieden sind und 0 oder 1 bedeuten,

sowie deren Stereoisomere, dadurch gekennzeichnet, daß man eine Verbindung der Formel II

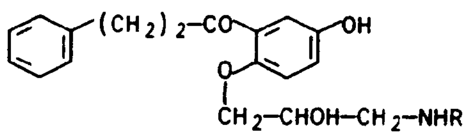
II,

worin R die angegebene Bedeutung hat, mit einem Glycerid der Formel III

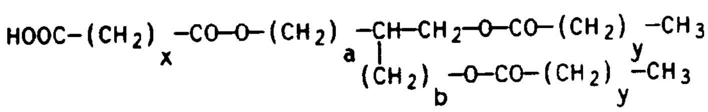
III,

worin x, y, a und b die angegebene Bedeutung besitzen, umsetzt.

3. Verbindungen der Formel I gemäß Anspruch 1 zur Verwendung bei der Bekämpfung von Krankheiten.

## Revendications

1. Glycérides de l'Hydroxypropafenon, de formule I

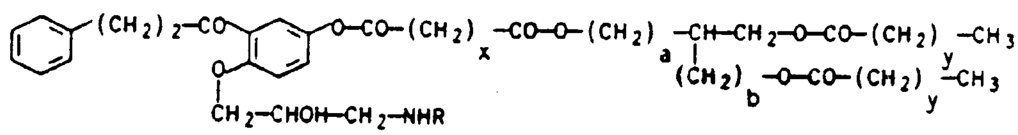 I,

dans laquelle

R représente un groupe n-propyle ou 1,1-diméthylpropyle,

x est égal à 2 ou 3,

y est égal à 12, 14, 16, 18 ou 20, et

a et b sont égaux chacun, indépendamment l'un de l'autre, à 0 ou 1,

et leurs stéréoisomères.

2. Procédé de préparation des glycérides de l'Hydroxypropafenon de formule I

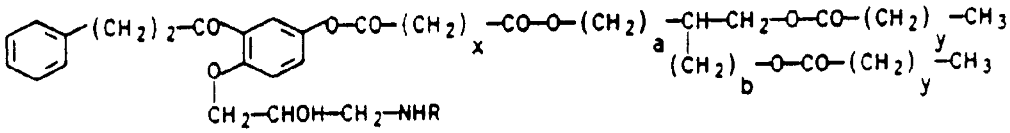 I,

dans laquelle

R représente un groupe n-propyle ou 1,1-diméthylpropyle,

x est égal à 2 ou 3,

y est égal à 12, 14, 16, 18 ou 20, et

a et b sont égaux chacun, indépendamment l'un de l'autre à 0 ou 1,

et de leurs stéréoisomères, caractérisé en ce que l'on fait réagir un composé de formule II

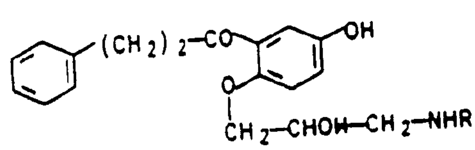 II,

dans laquelle R a les significations indiquées ci-dessus, avec un glycéride de formule III

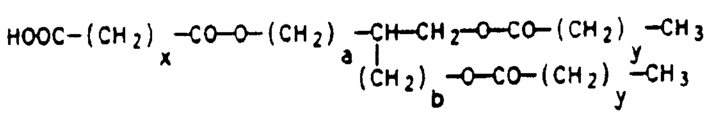 III,

dans laquelle x, y, a et b ont les significations indiquées ci-dessus.

3. Composés de formule I de la revendication 1, pour l'utilisation dans le traitement de maladies.

## Claims

1. A hydroxypropafenone glyceride of the formula I

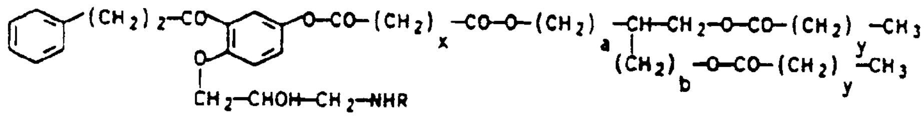

I,

where

R is n-propyl or 1,1-dimethylpropyl,

x is 2 or 3,

y is 12, 14, 16, 18 or 20, and

a and b differ from one another and are 0 or 1,

as well as the stereoisomers thereof.

2. A process for the preparation of a hydroxypropafenone glyceride of the formula I

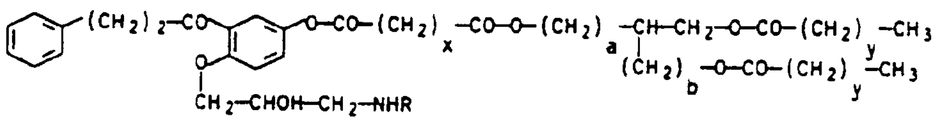

I,

where

R is n-propyl or 1,1-dimethylpropyl,

x is 2 or 3,

y is 12, 14, 16, 18 or 20, and

a and b differ from one another and are 0 or 1,

as well as the stereoisomers thereof, which comprises reacting a compound of the formula II

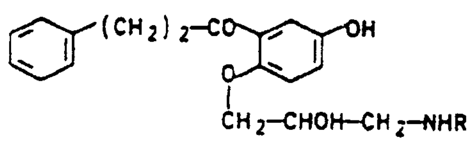

II,

where R has the stated meaning, with a glyceride of the formula III

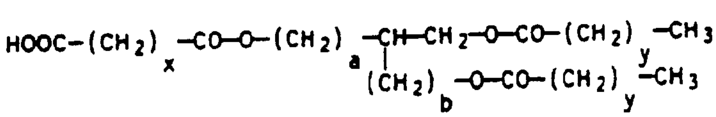

III,

where x, y, a and b have the stated meanings.

3. A compound of the formula I as claimed in claim 1 for use in controlling diseases.